Europäisches Patentamt

European Patent Office

Office européen des brevets

(11) Veröffentlichungsnummer: **0 616 197 A1**

## (12) EUROPÄISCHE PATENTANMELDUNG

(21) Anmeldenummer: **94103793.9**

(22) Anmeldetag: **11.03.94**

(51) Int. Cl.⁵: **G01D 18/00**, G01N 33/00

(30) Priorität: **19.03.93 DE 4308936**

(43) Veröffentlichungstag der Anmeldung:
**21.09.94 Patentblatt 94/38**

(84) Benannte Vertragsstaaten:
**AT BE CH DE ES FR GB IT LI**

(71) Anmelder: **SIEMENS AKTIENGESELLSCHAFT**
**Wittelsbacherplatz 2**
**D-80333 München (DE)**

(72) Erfinder: **Kimmig, Ludwig**
**Schumacherstrasse 17**
**D-76275 Ettlingen (DE)**
Erfinder: **Krause, Peter**
**Binger Strasse 11**
**D-76185 Karlsruhe (DE)**

(54) Verfahren zum Kalibrieren von Messgeräten und Messgerät mit einer Steuereinheit zur Durchführung des Verfahrens.

㉗ Zum Kalibrieren von Meßgeräten werden mehrere Kalibrierwerte in Zeitabständen gemessen. Mit einer vorgegebenen Zahl von jüngsten gemessenen Kalibrierwerten werden mittels einer nichtlinearen Funktion künftige Kalibrierwerte errechnet, mit denen Meßwerte korrigiert werden.

Hauptanwendungsgebiet sind Einstrahl-IR-Absorptions-Photometer.

EP 0 616 197 A1

Die Erfindung betrifft ein Verfahren zum Kalibrieren von Meßgeräten und ein Meßgerät mit einer Steuereinheit zur Durchführung des Verfahrens.

Zahlreiche Meßgeräte haben die Eigenschaft, daß sie von Zeit zu Zeit kalibriert werden müssen. Auch kann oft nach dem Einschalten erst nach einer längeren Anwärmphase, in der sich im Gerät ein Temperaturgleichgewicht einstellt, gemessen werden. Dies ist z. B. bei IR-Absorptions-Photometern bzw. nach deren Prinzip arbeitenden Auto-Abgastestern der Fall. Bedingt durch die Erwärmung des physikalischen Teils (Meßküvette, Detektor) nach dem Einschalten und durch Temperaturgradienten während des Betriebes verändern sich Empfindlichkeit und Nullpunkt. Im Falle eines Gasanalysators sind daher Messungen von Gaskonzentrationen ohne Korrekturmaßnahmen, wie Nullpunkt und Empfindlichkeitseinstellungen, nicht mit ausreichender Genauigkeit möglich. Dem Problem der temperaturbedingten Drift von Meßgeräten kann durch eine lange Anwärmphase oder durch aufwendige konstruktive Maßnahmen begegnet werden.

Der vorliegenden Erfindung liegt die Aufgabe zugrunde, ein Verfahren zum Kalibrieren von Meßgeräten, insbesondere von nach dem Prinzip eines IR-Absorptions-Photometers arbeitenden Gasanalysatoren zu schaffen, mit dem auch bei einfachem konstruktivem Aufbau des Meßgerätes Veränderungen von Nullpunkt und/oder der Empfindlichkeit zuverlässig kompensiert werden können. Eine weitere Aufgabe besteht darin, ein Meßgerät, insbesondere einen Gasanalysator, mit einer Steuereinheit zur Durchführung eines solchen Kalibrierverfahrens zu schaffen.

Erfindungsgemäß wird diese Aufgabe mit den in den Ansprüchen 1 und 7 angegebenen Maßnahmen gelöst.

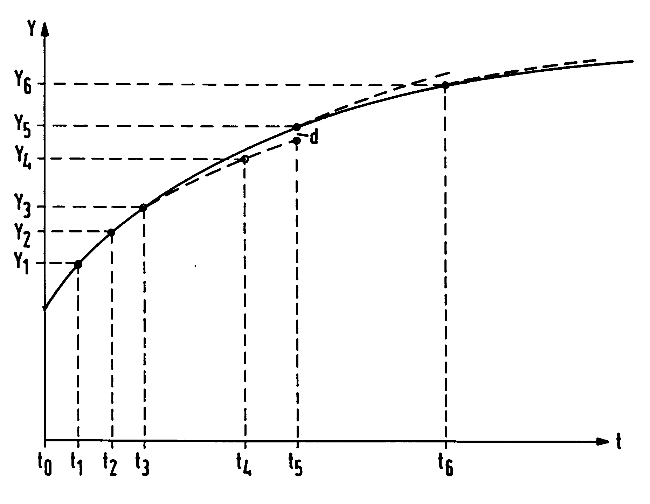

Anhand der Zeichnung werden im folgenden das erfindungsgemäße Verfahren sowie Ausgestaltungen und Weiterbildungen beschrieben und erläutert, und zwar für den Fall des Nullabgleichs eines Einstrahl-IR-Absorptions-Photometers, mit dem die Komponenten Kohlenwasserstoffe, Kohlenmonoxid und Kohlendioxid im Abgas eines Verbrennungsmotors gemessen werden.

Die Meßzelle des Photometers wird mit sogenanntem Nullgas gespült, einem Gas, das keine der zu messenden Komponenten enthält. In der Meßzelle wird daher von solchen Komponenten keine IR-Strahlung absorbiert; der Detektor gibt ein maximales Ausgangssignal ab. In der Figur ist der Verlauf des Ausgangssignals des Photometers bei mit Nullgas gespülter Meßzelle über die Zeit $t$ aufgetragen. Nach dem Einschalten im Zeitpunkt $t_0$ steigt das Ausgangssignal des Detektors zunächst steil an. In dieser Zeit ist eine Messung von Meßgaskomponenten und auch eine Nullpunktbestimmung nicht sinnvoll. Im Zeitpunkt $t_1$, z. B. nach einer Minute, wird zum ersten Mal eine Nullpunktmessung durchgeführt; es wird der Wert $Y_1$ erhalten. Zu zwei weiteren Zeitpunkten $t_2$, $t_3$ werden zwei Nullpunkte $Y_2$, $Y_3$ gemessen. Mit den drei gemessenen Werten wird der künftige Verlauf des Nullpunktes berechnet, und zwar mittels der Gleichung

$$Y = A + B \cdot t + C \cdot \frac{1}{t},$$

die als dem tatsächlichen Verlauf nahekommend ermittelt wurde. Der berechnete Verlauf ist mit einer gestrichelten Linie in die Figur eingetragen.

Nach der ersten Vorausberechnung der Nullpunkte wird mit den Messungen am Meßgas begonnen. Die folgenden Meßwerte werden mit den für die jeweilige Meßzeit berechneten Nullpunkt korrigiert. Wird z. B. im Zeitpunkt $t_4$ ein Wert M gemessen, dann wird er durch Subtraktion mit dem berechneten Nullpunkt $Y_4$ korrigiert.

Die Messungen der Nullpunkte erfolgen nun in größeren Abständen, z. B. von 15 min, in den Zeitpunkten $t_5$ und $t_6$. Für die Vorausberechnung der Nullpunkte, deren Verlauf wieder als gestrichelte Linie dargestellt ist, werden nach der Messung des Wertes $Y_5$ die zu den Zeiten $t_2$, $t_3$, $t_5$ gemessenen Werte $Y_2$, $Y_3$, $Y_5$ herangezogen. Im Zeitpunkt $t_6$ erfolgt dann die nächste Nullpunktmessung und die nächste Nullpunktberechnung, wobei wieder die drei jüngsten Nullpunktwerte $Y_3$, $Y_5$, $Y_6$ zur Bestimmung der Parameter A, B, C in der obigen Gleichung benutzt werden.

In der bisherigen Beschreibung wurde angenommen, daß in konstanten Zeitabständen die Nullpunkte gemessen werden und ihr Verlauf berechnet wird. Statt dessen können auch die Zeiten in Abhängigkeit von der Nullpunktänderung gewählt werden. Beispielsweise können die Zeiten in Abhängigkeit der Abweichung der gemessenen von den berechneten Werten verlängert oder verkürzt werden. Ist z. B. die Abweichung d im Zeitpunkt $t_5$ größer als ein vorgegebener Betrag, wird die nächste Messung früher erfolgen, als wenn die Abweichung kleiner als der vorgegebene Betrag ist. Man erreicht damit, daß die Nullpunkte nur so oft wie nötig gemessen werden, damit für die Messung des Meßgases möglichst viel Zeit zur Verfügung steht. Aus demselben Grunde kann man auch den Zeitpunkt für die jeweils nächste Nullpunktmessung aufgrund der berechneten Nullpunktsänderung festlegen. Die jeweils nächste Nullpunktmessung wird zu dem Zeitpunkt durchgeführt, zu dem die Differenz zwischen dem berechneten Nullpunkt und dem jeweils zuletzt gemessenen einen vorgegebenen Wert hat.

Das neue Verfahren wurde anhand des Nullpunktabgleichs eines Einstrahl-IR-Absorptions-Photometers beschrieben.

Prinzipiell kann es aber auch zur Nullpunkts- oder zur Empfindlichkeitseinstellung anderer Meßgeräte eingesetzt werden, wobei allerdings im allgemeinen eine andere Formel als die oben angegebene als Vorausberechnung verwendet werden muß.

Meßgeräte, die in der beschriebenen Weise kalibriert werden, sind mit einer Steuereinheit versehen, welche das beschriebene Verfahren in der gewünschten Modifikation ausführt, also insbesondere die Zeitpunkte der Nullpunktmessungen bestimmt, die Nullpunktmessung einleitet und den Nullpunkt berechnet.

**Patentansprüche**

1.  Verfahren zum Kalibrieren von Meßgeräten, **dadurch gekennzeichnet**, daß mehrere Kalibrierwerte ($Y_1$, $Y_2$, ... $Y_6$) in Zeitabständen gemessen werden, daß mit einer vorgegebenen Zahl von jüngsten gemessenen Kalibrierwerten mittels einer dem zeitlichen Verlauf der Kalibrierwerte angenäherten Funktion künftige Kalibrierwerte errechnet werden, mit denen die Meßwerte korrigiert werden.

2.  Verfahren nach Anspruch 1, **dadurch gekennzeichnet**, daß die Zeitabstände, in denen die Kalibrierwerte gemessen werden, entsprechend der Differenz zwischen gemessenen und errechneten Kalibrierwerten bestimmt werden.

3.  Verfahren nach Anspruch 1 oder 2, **dadurch gekennzeichnet,** daß die Zeitabstände, in denen die Kalibrierwerte gemessen werden, entsprechend der zeitlichen Änderung der Kalibrierwerte bestimmt werden.

4.  Verfahren nach Anspruch 1, **dadurch gekennzeichnet,** daß in vorgegebenen Zeitabständen die Kalibrierwerte gemessen werden.

5.  Verfahren nach einem der Ansprüche 1 bis 4, **dadurch gekennzeichnet,** daß die Kalibrierwerte Nullpunkte sind und mit dem für den jeweiligen Meßzeitpunkt errechneten Kalibrierwert der Nullabgleich des Meßgerätes durchgeführt wird.

6.  Verfahren nach Anspruch 5, **dadurch gekennzeichnet**, daß bei einem IR-Absorptions-Photometer, insbesondere einem Einstrahl-Photometer, die Nullpunkte nach der Formel

$$Y = A + B \cdot t + C \cdot \frac{1}{t}$$

errechnet werden, worin t die Zeit und A, B, C Parameter sind, die aufgrund von je drei gemessenen Nullpunkten errechnet werden.

7.  Meßgerät mit einer Steuereinheit zum Durchführen eines der Verfahren nach den Ansprüchen 1 bis 6.

Europäisches
Patentamt

**EUROPÄISCHER RECHERCHENBERICHT**

Nummer der Anmeldung

EP 94 10 3793

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | PATENT ABSTRACTS OF JAPAN vol. 14, no. 179 (P-1034) 10. April 1990 & JP-A-02 028 516 (ANRITSU CORP) 30. Januar 1990 * Zusammenfassung * --- | 1,4,5 | G01D18/00 G01N33/00 |
| A | DATABASE WPI Week 9242, Derwent Publications Ltd., London, GB; AN 92-347441 & SU-A-1 673 960 (AS BELO PHYS INST) 30. August 1990 * Zusammenfassung * ----- | 6 | |
| | | | **RECHERCHIERTE SACHGEBIETE (Int.Cl.5)** G01D G01N |

Der vorliegende Recherchenbericht wurde für alle Patentansprüche erstellt

| Recherchenort | Abschlußdatum der Recherche | Prüfer |
|---|---|---|
| DEN HAAG | 7. Juni 1994 | Lut, K |

EPO FORM 1503 03.82 (P04C03)